# EUROPEAN PATENT APPLICATION

(11) **EP 4 066 822 A1**
(43) Date of publication of application: **05.10.2022**
(21) Application number: 22154078.4
(22) Date of filing: 16.08.2016
(51) Int. Cl.: A61K 9/48, A61K 31/395, A61K 31/436, A61K 47/38, A61K 31/4353, A61K 45/06

(54) **PHARMACEUTICAL FORMULATIONS**

(30) Priority: 19.08.2015 US 201562207324 P
(62) Divisional of application: 16837688.7
(71) Applicant: Vivus, Inc., Campbell CA 95008 (US)
(72) Inventor: BANAIT, Narinder S., Saratoga, 95070 (US); GU, Leo, Saratoga, 95070 (US)
(74) Representative: Cooley (UK) LLP

(57) **Abstract**

The present invention provides compositions and methods for the treatment or prevention of pulmonary hypertension comprising administering an ascomycin, or a pharmaceutically acceptable salt, solvate, analog, or prodrug thereof to the patient with pulmonary hypertension. Described herein are liquid formulations which deliver an ascomycin. The liquid formulation can be placed in a soft gelatin capsule.

## Description

### FIELD OF INVENTION

The present disclosure relates to methods for the treatment or prevention of pulmonary hypertension. In particular, the present disclosure relates to modulators of bone morphogenetic protein receptor type II (BMPR2), pharmaceutical formulations thereof and their use, alone or in combination with one or more additional agents, for treating and/or preventing various diseases, wherein an increase in the concentration of bone morphogenetic proteins (BMP) might be desirable.

### BACKGROUND

Tacrolimus, also known as FK-506 or FR-900506, is a macrolide agent that inhibits T-lymphocyte activation through a process that is thought to involve it binding to an intracellular protein, FKBP-12. A hydrophobic complex of tacrolimus-FKBP-12, calcium, calmodulin, and calcineurin is then formed and the phosphatase activity of calcineurin inhibited. This effect may prevent the dephosphorylation and translocation of nuclear factor of activated T-cells (NF-AT), a nuclear component thought to initiate gene transcription for the formation of lymphokines. The resulting inhibition of T-lymphocyte activation leads to immunosuppression.

Tacrolimus appears as white crystals or crystalline powder that is practically insoluble in water, freely soluble in ethanol and very soluble in methanol and chloroform. Absorption of tacrolimus is rapid, variable, and incomplete from the gastrointestinal tract (Harrison's Principles of Internal Medicine, 14th edition, 1998, McGraw Hill, 14, 20, 21, 64-67) and it is metabolized primarily by the CYP3A4 isoenzyme in the small intestine (gut wall) and liver.

Tacrolimus is differentially absorbed in different regions of the gastrointestinal tract, being optimally absorbed from the small intestine, with ileum and colonic absorption efficiency dropping to half that observed for the small intestine. The mean bioavailability of the oral dosage form is about 27%, (range 5 to 65%). The volume of distribution (VoID) based on plasma is 5 to 65 L/kg of body weight, and is much higher than the VoID based on whole blood concentrations, the difference reflecting the binding of tacrolimus to red blood cells. Whole blood concentrations may be 12 to 67 times the plasma concentrations. Protein binding is high (75 to 99%). The half-life for distribution is 0.9 hour, and the time to peak concentration is 0.5 to 4 hours after oral administration.

Tacrolimus is currently available in topical, intravenous and oral dosage forms. The topical formulation is commercially known as Ptotopic^{®}. The topical ointment is sold in 2 strengths, 0.1% for adults and teenagers who are 16 and older, and 0.03% for children over the age of 2. The formulation contains tacrolimus as the active ingredient, and contains mineral oil, paraffin, white petrolatum, white wax and propylene carbonate as inactive ingredients. Topical tacrolimus is prescribed for the treatment of eczema. Another topical for the treatment of eczema is Elidel^{®} that contains pimecrolimus as the active agent.

The intravenous dosage form contains tacrolimus, polyoxyethylene hydrogenated castor oil, and dehydrated alcohol to give a clear colorless solution. The solution is diluted with saline solution prior to infusion. Immediate release capsule formulation of tacrolimus is commercially known as Prograf^{®}. However, the immediate release formulation of the drug is poorly tolerated and provides a variable and/or low bioavailability.

An extended release tablet formulation of tacrolimus manufactured using the MeltDose processing technology is known. Tacrolimus is dissolved in high molecular weight polyethylene glycol (PEG6000) and poloxamer 188, and sprayed on lactose using fluid bed granulation. The granules are sieved to obtain a desired size, mixed with extra granular excipients and compressed into tablets. The tablets are then coated with hypromellose as the release control polymer. These tablets have a flatter PK profile.

An extended release once-daily capsule formulation of tacrolimus is also known. The formulation process consists of tacrolimus dissolved in dehydrated ethanol, and being granulated with ethylcellulose, hypromellose and lactose monohydrate. The hypromellose system modifies the drug release profile by forming a polymer gel layer and the ethylcellulose diffusion matrix system modifies the release profile by controlling water penetration and thus drug release. The resulting paste undergoes drying and sizing to produce intermediate granules. The granules are then mixed with lactose monohydrate and magnesium stearate and that mixture is filled into capsules. The formulation results in dissolution of 90% drug release at 6 to 12 hours. One potential problem with this once-daily product results in an initial spike in the drug plasma concentration, with the potential to cause unwanted side effects.

There is, therefore, a need for an improved composition of tacrolimus that will have a favorable PK profile.

### SUMMARY

The present invention provides compositions, methods, and pharmaceutical formulations for the treatment of pulmonary hypertension, in particular pulmonary arterial hypertension.

In one aspect, the present invention describes a method of treating or preventing pulmonary hypertension in a patient in need thereof, the method comprising administering a therapeutically effective amount of a compound that increases BMPR2 signaling (BMPR2 activator) to the patient with pulmonary hypertension or a condition related thereto. The subject can be a mammal, such as a human. The BMPR2 activator can be an ascomycin or a pharmaceutically acceptable salt, solvate, analog or prodrug thereof.

In another aspect, the present invention describes a soft gelatin capsule formulation comprising a shell and a liquid fill material wherein the liquid fill material comprises an ascomycin class compound, or a pharmaceutically acceptable salt, solvate, analog, or prodrug thereof, dissolved in a solvent. The ascomycin class compound can be tacrolimus (FK-506), ascomycin (FK-520), pimecrolimus (33-epi-chloro-33-desoxy-ascomycin), ABT-281, SDZ 281-240, desmethyl acomycin (FK-523), (prolytacrolimus(FK-525), or combinations thereof.

These and other aspects of the present invention will become evident upon reference to the following detailed description

### DETAILED DESCRIPTION

### I. Definitions

Unless otherwise stated, the following terms used in this application, including the specification and claims, have the definitions given below. It must be noted that, as used in the specification and the appended claims, the singular forms "a," "an" and "the" include plural referents unless the context clearly dictates otherwise. Definition of standard chemistry terms may be found in reference works, including Carey and Sundberg (2004) "Advanced Organic Chemistry 4^{rd} Ed." Vols. A and B, Springer, New York. The practice of the present invention will employ, unless otherwise indicated, conventional methods of mass spectroscopy, protein chemistry, biochemistry, and pharmacology, within the skill of the art.

The term "modulator" means a molecule that interacts with a target. The interactions include, but are not limited to, agonist, antagonist, and the like, as defined herein.

The tenn "agonist" means a molecule such as a compound, a drug, an enzyme activator or a hormone that enhances the activity of another molecule or the activity of the target receptor.

The term "antagonist" means a molecule such as a compound, a drug, an enzyme inhibitor, or a hormone, that diminishes or prevents the action of another molecule or the activity of the target receptor.

The terms "effective amount" or "pharmaceutically effective amount" refer to a sufficient amount of the agent to provide the desired biological result without an unacceptable toxic effect. That result can be reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in a disease. An appropriate "effective" amount in any individual case may be determined by one of ordinary skill in the art using routine experimentation.

As used herein, the terms "treat" or "treatment" are used interchangeably and are meant to ameliorating the disease or disorder (i.e., arresting or reducing the development of the disease or at least one of the clinical symptoms thereof). In one embodiment "treating" or "treatment" refers to ameliorating at least one symptoms of the disease. In another embodiment, "treating" or "treatment" refers to inhibiting the disease or disorder, either physically (e.g., stabilization of a discernible symptom), physiologically, (e.g., stabilization of a physical parameter), or both.

By "pharmaceutically acceptable" or "pharmacologically acceptable" is meant a material which is not biologically or otherwise undesirable, i.e., the material may be administered to an individual without causing any undesirable biological effects or interacting in a deleterious manner with any of the components of the composition in which it is contained.

As used herein, the term "mammal subject" encompasses any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like.

The term "pharmaceutically acceptable salt" of a compound means a salt that is pharmaceutically acceptable and that possesses the desired pharmacological activity of the parent compound. Such salts, for example, include:
(1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 2-naphthalenesulfonic acid, 4-methylbicyclo-[2.2.2]oct-2-ene-1-carboxylic acid, glucoheptonic acid, 44'-methylenebis-(3-hydroxy-2-ene-1 -carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like;
(2) salts formed when an acidic proton present in the parent compound either is replaced by a metal ion, e.g., an alkali metal ion, an alkaline earth ion, or an aluminum ion; or coordinates with an organic base. Acceptable organic bases include ethanolamine, diethanolamine, triethanolamine, tromethamine, N-methylglucamine, and the like. Acceptable inorganic bases include aluminum hydroxide, calcium hydroxide, potassium hydroxide, sodium carbonate, sodium hydroxide, and the like. It should be understood that a reference to a pharmaceutically acceptable salt includes the solvent addition forms or crystal forms thereof, particularly solvates or polymorphs. Solvates contain either stoichiometric or non-stoichiometric amounts of a solvent, and are often formed during the process of crystallization. Hydrates are formed when the solvent is water, or alcoholates are formed when the solvent is alcohol. Polymorphs include the different crystal packing arrangements of the same elemental composition of a compound. Polymorphs usually have different X-ray diffraction patterns, infrared spectra, melting points, density, hardness, crystal shape, optical and electrical properties, stability, and solubility. Various factors such as the recrystallization solvent, rate of crystallization, and storage temperature may cause a single crystal form to dominate.

The term "optional" or "optionally" means that the subsequently described event or circumstance may or may not occur, and that the description includes instances where the event or circumstance occurs and instances where it does not.

All publications, patents and patent applications cited herein, whether *supra* or *infra*, are hereby incorporated by reference in their entirety.

### II. DESCRIPTION OF THE INVENTION

The compositions and methods of the present invention increase BMPR2 pathway signaling. Thus, the present invention provides compositions and methods for the prevention or treatment of a BMPR2 pathway mediated condition or disease. The BMPR2 pathway is a critically important pathway, the expression of which is reduced in patients with pulmonary arterial hypertension (PAH). Therefore, increasing BMPR2 signaling in patients with PAH can prevent or reverse disease.

### Active Agent

In particular, the present invention provides for the use of a compound for the treatment of PAH selected from: idiopathic PAH; familial PAH; PAH associated with a collagen vascular disease selected from: scleroderma, CREST syndrome, systemic lupus erythematosus (SLE), rheumatoid arthritis, Takayasu's arteritis, polymyositis, and dermatomyositis; PAH associated with a congenital heart disease selected from: atrial septic defect (ASD), ventricular septic defect (VSD) and patent ductus arteriosus in an individual; PAH associated with portal hypertension; PAH associated with HIV infection; PAH associated with ingestion of a drug or toxin; PAH associated with hereditary hemorrhagic telangiectasia; PAH associated with splenectomy; PAH associated with significant venous or capillary involvement; PAH associated with pulmonary veno-occlusive disease (PVOD); and PAH associated with pulmonary capillary hemangiomatosis (PCH).

In one aspect, compositions and methods of treating or preventing pulmonary hypertension are described comprising administering a therapeutically effective amount of an active agent that is an ascomycin class compound (e.g., ascomycin) or a pharmaceutically acceptable salt, solvate, analog, or prodrug thereof. The ascomycin class compound is hereafter referred to as active agent. An ascomycin class compound is a macrolactam having a macrolide lactone structure. The ascomycin class compound can be tacrolimus, ascomycin, pimecrolimus (33-epi-chloro-33-desoxy-ascomycin), ABT-281, SDZ 281-240, FK523 (desmethyl acomycin), FK525 (prolytacrolimus), or a pharmaceutically acceptable salt, solvate, analog, or prodrug thereof.

In one aspect, the ascomycin class compound can be tacrolimus. Tacrolimus, also referred to as FK-506 or FR-900506, has a chemical name [3S-[3R^{∗}[E(1S^{∗},3S^{∗},4S^{∗})], 4S^{∗},5R^{∗},8S^{∗},9E,12R^{∗},14R^{∗},15S^{∗},16R^{∗},18S^{∗},19S^{∗},26- aR^{∗}]]-5,6,8,11,12,13,14,15,16,17,18,19,24,25,26a-hexadecahydro-5,19-dihydroxy-3-[2-(4-hydroxy-3-methoxycyclohexyl)-1-methylethenyl]-14,16-dimethoxy-4,10,12,18-tetramethyl-8-(2-propenyl)-15,19-epoxy-3H-pyrido[2,t-c][1,4]oxazacyclotricosine-1,7,20,21(4H,23H)-tetrone, monohydrate, having the formula C₄₄H₆₉NO₁₂, and has the structure shown below: 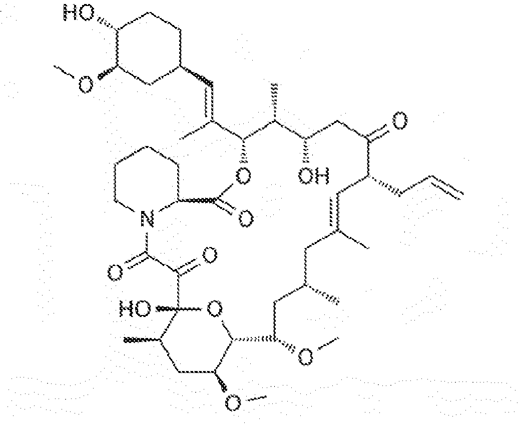 The preparation of tacrolimus is described in EP-A-0 184 162 and analogues of tacrolimus are disclosed U.S. Patent No. 6,387,918.

In another aspect, the ascomycin class compound can be the compound ascomycin, also known as FK520, with the IUPAC name of (3S,4R,5S,8R,9E,12S,14S,15R,16S,18R,19R,26aS)-8-ethyl-5,19-dihydroxy-3-{(1E)-1-[(1R,3R,4R)-4-hydroxy-3-methoxycyclohexyl]prop-1-en-2-yl}-14,16-dimethoxy-4,10,12,18-tetramethyl-5,6,8,11,12,13,14,15,16,17,18,19,24,25,26,26a-hexadecahydro-3H-15,19-epoxypyrido[2,1-c][1,4]oxazacyclotricosine- 1,7,20,21(4H,23H)-tetrone, having the formula C₄₃H₆₉NO₁₂, and the structure shown below:

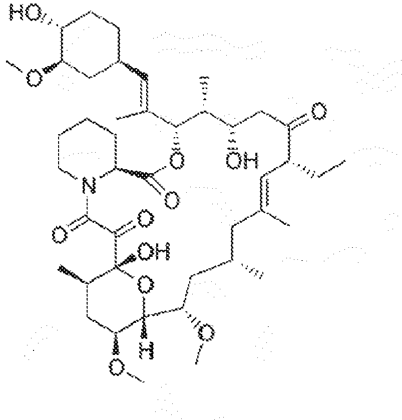

The present invention also provides prodrugs of an ascomycin and its analogues wherein the prodrug converts *in vivo* to ascomycin and its analogues. A prodrug is an active or inactive compound that is modified chemically through *in vivo* physiological action, such as hydrolysis, metabolism and the like, into a compound of this invention following administration of the prodrug to a subject. The suitability and techniques involved in making and using pro-drugs are well known by those skilled in the art. Prodrugs can be conceptually divided into two non-exclusive categories, bioprecursor prodrugs and carrier prodrugs. See The Practice of Medicinal Chemistry, Ch. 31-32 (Ed. Wermuth, Academic Press, San Diego, Calif., 2001). Generally, bioprecursor prodrugs are compounds, which are inactive or have low activity compared to the corresponding active drug compound that contain one or more protective groups and are converted to an active form by metabolism or solvolysis. Both the active drug form and any released metabolic products should have acceptably low toxicity.

Exemplary prodrugs are, for example, esters of free carboxylic acids and S-acyl derivatives of thiols and O-acyl derivatives of alcohols or phenols, wherein acyl has a meaning as defined herein. Suitable prodrugs are often pharmaceutically acceptable ester derivatives convertible by solvolysis under physiological conditions to the parent carboxylic acid, e.g., lower alkyl esters, cycloalkyl esters, lower alkenyl esters, benzyl esters, mono- or di-substituted lower alkyl esters. In addition, amines have been masked as arylcarbonyloxymethyl substituted derivatives which are cleaved by esterases in vivo releasing the free drug and formaldehyde (Bundgaard, J. Med. Chem. 2503 (1989)). Moreover, drugs containing an acidic NH group, such as imidazole, imide, indole and the like, have been masked with N-acyloxymethyl groups (Bundgaard, Design of Prodrugs, Elsevier (1985)). Hydroxy groups have been masked as esters and ethers. EP 039,051 (Sloan and Little) discloses Mannich-base hydroxamic acid prodrugs, their preparation and use.

Any compound given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds as defined above include isotopes of hydrogen, carbon, nitrogen, oxygen, phosphorous, fluorine, and chlorine, such as ²H, ³H, ¹¹C, ¹³C, ¹⁴C, ¹⁵N, ⁸F, ³¹P, ³²P, ³⁵S, ³⁶Cl, ¹²⁵I respectively. Isotopically labeled compounds of this invention and prodrugs thereof can generally be prepared by carrying out the synthetic procedures by substituting a readily available isotopically labeled reagent for a non-isotopically labeled reagent. Isotopically-labeled compounds can generally be prepared by conventional techniques known to those skilled in the art using an appropriate isotopically-labeled reagents in place of the non-labeled reagent previously employed.

The compounds disclosed above, in free form, may be converted into salt form, and vice versa, in a conventional manner understood by those skilled in the art. The compounds in free or salt form can be obtained in the form of hydrates or solvates containing a solvent used for crystallization. The compounds can be recovered from reaction mixtures and purified in a conventional manner. Pharmaceutically acceptable solvates in accordance with the invention include those wherein the solvent of crystallization may be isotopically substituted, e.g. D₂O, d₆-acetone, d₆-DMSO.

An ascomycin, or a pharmaceutically acceptable salt, solvate, analog, or prodrug thereof, that increases BMPR2 signaling can be administered to a patient for the treatment or prevention of PAH. Treatment or prevention of PAH as used herein encompasses one or more of the following:
(a) adjustment of one or more hemodynamic parameters towards a more normal level, for example lowering mean PAP or PVR, or raising PCWP or LVEDP, versus baseline;
(b) improvement of pulmonary function versus baseline, for example increasing exercise capacity, illustratively as measured in a test of 6-minute walking distance (6MWD), or lowering Borg dyspnea index (BDI);
(c) improvement of one or more quality of life parameters versus baseline, for example an increase in score on at least one of the SF-36^{™} health survey functional scales;
(d) general improvement versus baseline in the severity of the condition, for example by movement to a lower WHO functional class;
(e) improvement of clinical outcome following a period of treatment, versus expectation in absence of treatment (e.g., in a clinical trial setting, as measured by comparison with placebo), including improved prognosis, extending time to or lowering probability of clinical worsening, extending quality of life (e.g., delaying progression to a higher WHO functional class or slowing decline in one or more quality of life parameters such as SF-36^{™} health survey parameters), and/or increasing longevity; and/or
(f) adjustment towards a more normal level of one or more molecular markers that can be predictive of clinical outcome, such as plasma concentrations of bone morphogenetic protein (BMP), cardiac troponin T (cTnT), NT-proBNP, or B-type natriuretic peptide (BNP)).

An ascomycin, or a pharmaceutically acceptable salt, solvate, analog, or prodrug thereof can be administered in a therapeutically effective amount sufficient to provide any one or more of the effects mentioned above. Preferably the amount administered does not exceed an amount causing an unacceptable degree of adverse side effects. The therapeutically effective amount can vary depending on the compound, the particular pulmonary hypertension condition to be treated, the severity of the condition, body weight and other parameters of the individual subject, and can be readily established without undue experimentation by the physician or clinician based on the disclosure herein. Typically, a therapeutically effective amount will be found in the range of about 0.1 to about 25 mg/day, for example about 0.5 to about 15 mg/day, about 1 to about 10 mg/day, or about 0.5, about 1, about 1.5, about 2, about 2.5, about 3, about 3.5, about 4, about 4.5, about 5, about 6, about 7, about 8, about 9 or about 10 mg/day. The therapeutically effective amount can be administered each day, for example in individual doses administered once, twice, or three or more times a day. The therapeutically effective amount can be administered once each day, once every other day, or once every third day.

For example, if the compound to increase BMPR2 signaling is ascomycin or a phannaceutically acceptable solvate, salt, analog, or prodrug thereof, it can be administered at a dose and regimen that provides ascomycin whole blood concentration of about 0.05 ng/ml to about 30 ng/ml, such as about 0.1 ng/mL to about 0.5 ng/mL, about 0.15 ng/mL to about 0.3 ng/mL or about 0.1-0.2 ng/mL. In part because ascomycin is metabolized by the cytochrome P450 system, the exact dosing may vary between patients. Ascomycin or a pharmaceutically acceptable solvate, salt, analog, or prodrug thereof can be administered once, twice, or three or more times a day. In one aspect of the invention, the goal is to reach a whole blood level of about 0.2 ng/mL to about 30 ng/mL. In this case, an initial dose of 0.001 mg/kg day to 0.01 mg/kg day (e.g., 0.002 mg kg /day to 0.05 mg/kg/day may be sufficient, and the does can be up-titrated according to the measured ascomycin whole blood level. In particular cases, the ascomycin may reach a whole blood concentration as low as 0.1-0.2 ng/ml (e.g., 0.10 to 0.12, 0.12 to 0.14, 0.14 to 0.16, 0.16 to 0.18 or 0.18 to 0.20), however whole blood a concentration in the range of 0.2 to 30 ng/ml, e.g., 0.2, 0.5, 1 and 2 ng/ml may be acceptable. In particular cases, ascomycin can reach a whole blood concentration of <1.0, 1.5-2.5, or 3-5 ng/ml.

The active agent to increase BMPR2 signaling can be administered in monotherapy. Alternatively, the compound to increase BMPR2 signaling can be administered in combination therapy with one or more other active agent effective for the treatment of the pulmonary hypertension condition or a condition related thereto. When a second or more active agent is administered concomitantly, one of skill in the art can readily identify a suitable dose for any particular second active agent from publicly available information in printed or electronic form, for example on the internet. Illustratively and without limitation, the active agent to increase BMPR2 signaling can be administered with a second active agent comprising at least one drug selected from the group consisting of prostanoids, phosphodiesterase inhibitors, especially phosphodiesterase-5 (PDE5) inhibitors, endothelin receptor antagonists (ERAs), prostacyclin receptor (IP receptor) agonist, soluble guanylate cyclase stimulator, calcium channel blockers, diuretics, anticoagulants, nitric oxide, oxygen and combinations thereof.

In one aspect, an ascomycin, or a pharmaceutically acceptable salt, solvate, analog, or prodrug thereof can be administered alone or in combination with other active compounds. Thus, compounds that increase the signaling of the BMPR2 pathway can further be combined with other compounds that increase vasodilation such as compounds that target endothelin (Tracleer^{®}, Opsumit^{®}, and Letairis^{®}), nitric oxide/PDE-5 (Revatio^{®}, Adcirca^{®}, avanafil, lodenafil, mirodenafil, udenafil, and zaprinast), prostacyclin (Remodulin^{®}, Tyvaso^{®}, and Flolan^{®}), prostacyclin receptor agonists (selexipag, and APD811), soluble guanylate cyclase (Riociguat^{®}), and the like. Thus, the combined compounds can become more effective agents for the treatment of PAH, and may provide additive or synergistic results from the combined use of the compounds that increase the signaling of the BMPR2 pathway with compounds that target other pathways.

Examples of drugs useful in combination therapy are classified and presented in several lists below. Some drugs are active at more than one target; accordingly certain drugs may appear in more than one list. Use of any listed drug in a combination is contemplated herein, independently of its mode of action.

A suitable prostanoid can be illustratively selected from the following list: beraprost, cicaprost, epoprostenol, iloprost, NS-304, PGE₁ prostacyclin, and treprostinil.

A suitable PDE5 inhibitor can illustratively be selected from the following list: sildenafil, tadalafil, vardenafil, avanafil, lodenafil, mirodenafil, udenafil, and zaprinast.

A suitable ERA other than ambrisentan can illustratively be selected from the following list: atrasentan, ambrisentan, BMS 193884, bosentan, CI-1020, darusentan, S-0139 SB-209670, sitaxsentan, TA-0201, tarasentan, TBC-3711, VML-588, and ZD-1611.

A suitable calcium channel blocker can illustratively be selected from the following list: Aryklalkylamines: bepridil, clentiazem, diltiazem, fendiline, gallopamil, mibefradil, prenylamine, semotiadil, terodiline, and verapamil; Dihydropyridine, derivatives: amlodipine, aranidipine, barnidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, and NZ 105; Piperazine derivatives: cinnarizine, dotarizine, flunarizine, lidoflazine, and lomerizine; and Unclassified: bencyclane, etafenone, fantofarone, monatepil, perhexiline. Particularly suitable calcium channel blockers include amlodipine, diltiazern, felodipine, isradipine, nicardipine, nifedipine, nisoldipine, verapamil and combinations thereof.

A suitable diuretic can illustratively be selected from the following list: Organomercurials: chlormerodrin, chlorothiazide, chlorthalidone, meralluride, mercaptomerin, sodium mercumatilin, sodium mercurous, and chloride mersalyl; Purines: pamabrom, protheobromine, and theobromine; Steroids: canrenone, oleandrin, and spironolactone; Sulfonamide derivatives: acetazolamide, ambuside, azosemide, bumetanide, butazolamide, chloraminophenamide, clofenamide, clopamide, clorexolone, disulfamide, ethoxzolamide, furosemide, metruside, methazolarnide, piretanide, torsemide, triparnide, and xipamide; Thiazides and analogs: althiazide, bendroflumethiazide, benzthiazide, benzylhydrochlorothiazide, buthiazide, chlorthalidone, cyclopenthiazide, cyclothiazide, ethiazide, fenquizone, hydrochlorothiazide, hydroflumethiazide, indapamide, methyclothiazide, metolazone, paraflutizide, polythiazide, quinethazone, teclothiazide, and trichlormethiazide; Uracils: aminometradine; Unclassified: amiloride, Biogen BG 9719, chlorazanil, ethacrynic acid, etozolin, isosorbide, Kiowa Hakko KW 3902, mannitol, muzolimine, perhexiline, Sanofi-Aventis SR 121463, ticrynafen, triamterene, and urea. In some embodiments, the diuretic if present comprises a thiazide or loop diuretic. Thiazide diuretics are generally not preferred where the patient has a complicating condition such as diabetes or chronic kidney disease, and in such situations a loop diuretic can be a better choice. Particularly suitable thiazide diuretics include chlorothiazide, chlorthalidone, hydrochlorothiazide, indapamide, metolazone, polythiazide and combinations thereof. Particularly suitable loop diuretics include bumetanide, furosemide, torsemide and combinations thereof.

A suitable anticoagulant can illustratively be selected from the following list: acenocoumarol, ancrod, anisindione, bromindione, clorindione, coumetarol, cyclocumarol, dextran sulfate, sodium dicumarol, diphenadione, ethyl biscoumacetate, ethylidene dicoumarol, fluindione, heparin, hirudin, lyapolate, sodium pentosan, polysulfate phenindione, phenprocoumon, phosvitin, picotamide, tioclomarol, and warfarin.

Where the pulmonary hypertension condition is associated with an underlying disease (for example CTD, HIV infection, COPD or ILD), the active agent to increase BMPR2 signaling can optionally be administered in combination therapy with one or more drugs targeting the underlying condition.

When the active agent to increase BMPR2 signaling is used in combination therapy with one or more drugs, the active agent and at least one drug can be administered at different times or at about the same time (at exactly the same time or directly one after the other in any order). The active agent and the second active drug can be formulated in one dosage form as a fixed-dose combination for administration at the same time, or in two or more separate dosage forms for administration at the same or different times.

Separate dosage forms can optionally be co-packaged, for example in a single container or in a plurality of containers within a single outer package, or co-presented in separate packaging ("common presentation"). As an example of co-packaging or common presentation, a kit is contemplated comprising, in separate containers, active agent to increase BMPR2 signaling and at least one drug useful in combination with the active agent. In another example, the active agent and the at least one drug useful in combination therapy with the active agent are separately packaged and available for sale independently of one another, but are co-marketed or co-promoted for use according to the invention. The separate dosage forms can also be presented to a patient separately and independently, for use according to the invention.

### Soft Gelatin Formulation

The compounds described above are preferably used to prepare a medicament, such as by formulation into pharmaceutical compositions for administration to a subject using techniques generally known in the art. A summary of such pharmaceutical compositions may be found, for example, in Remington's Pharmaceutical Sciences (the latest edition) Mack Publishing Co., Easton, PA. The compounds of the invention can be used singly or as components of mixtures. Preferred forms of the compounds are those for systemic administration as well as those for topical or transdermal administration. Formulations designed for timed release are also with the scope of the invention. Formulation in unit dosage form is also preferred for the practice of the invention.

In unit dosage form, the formulation is divided into unit doses containing appropriate quantities of one or more compound. The unit dosage may be in the form of a package containing discrete quantities of the formulation. Non-limiting examples are packeted tablets or capsules, and powders in vials or ampoules.

The compounds of the invention may be labeled isotopically (e.g. with a radioisotope) or by another other means, including, but not limited to, the use of chromophores or fluorescent moieties, bioluminescent labels, or chemiluminescent labels. The compositions may be in conventional forms, either as liquid solutions or suspensions, solid forms suitable for solution or suspension in a liquid prior to use, or as emulsions. Suitable excipients or carriers are, for example, water, saline, dextrose, glycerol, alcohols, aloe vera gel, allantoin, glycerin, vitamin A and E oils, mineral oil, propylene glycol, PPG-2 myristyl propionate, and the like. Of course, these compositions may also contain minor amounts of nontoxic, auxiliary substances, such as wetting or emulsifying agents, pH buffering agents, and so forth.

Methods for the preparation of compositions comprising the compounds of the invention include formulating the derivatives with one or more inert, pharmaceutically acceptable carriers to form either a solid or liquid. Solid compositions include, but are not limited to, powders, tablets, dispersible granules, capsules, cachets, and suppositories. Liquid compositions include solutions in which a compound is dissolved, emulsions comprising a compound, or a solution containing liposomes, micelles, or nanoparticles comprising a compound as disclosed herein.

A carrier of the invention can be one or more substances which also serve to act as a diluent, flavoring agent, solubilizer, lubricant, suspending agent, binder, or tablet disintegrating agent. A carrier can also be an encapsulating material.

For oral administration, the pharmaceutical composition can be in the form of, for example, a tablet, capsule, a soft gelatin (softgel) capsule, a hard gelatin capsule, suspension or liquid. The pharmaceutical composition is preferably made in the form of a dosage unit containing a particular amount of the active ingredient. Examples of such dosage units are tablets, hardgel capsules or softgel capsules.

Hard gelatin capsules can contain the compounds of the invention in combination with a solid, pulverulent carrier, such as, for example, lactose, saccharose, sorbitol, mannitol, potato starch, corn starch, amylopectin, cellulose derivatives, or gelatin.

Soft gelatin capsules can be prepared in which capsules contain the compounds of the invention and/or non-aqueous, and/or water miscible solvents such as polyethylene glycol and the like. Hydrophilic solvents compatible with softgel capsules can include PEG400, PEG800, ethanol, glycerin, PPG, polysorbates, povidone (PVP), and the like containing up to about 5-8% water. The softgel capsules can optionally contain a buffer, a co-solvent, a lipophilic surfactant, a hydrophilic surfactant, a plasticizer, a bioavailability enhancer, or a fatty acid.

### Solubilization of Therapeutic Agents

One composition or liquid formulation that may be used is a composition or liquid formulation in which the active agent is dissolved in a solvent component. Generally, any solvent which has the desired effect may be used in which the therapeutic agent dissolves. The solvent can be aqueous or non-aqueous. An "aqueous solvent" is a solvent that contains at least about 50% water.

Solvents for use in the liquid formulations can be determined by a variety of methods known in the art, including but not limited to (1) theoretically estimating their solubility parameter values and choosing the ones that match with the therapeutic agent, using standard equations in the field; and (2) experimentally determining the saturation solubility of therapeutic agent in the solvents, and choosing the ones that exhibit the desired solubility.

Generally, any concentration of solubilized active agent that has the desired effect can be used. The solvent component may be a single solvent or may be a mixture of solvents. Solvents and types of solutions are well known to those in drug delivery technologies. See for example, Remington: The Science and Practice of Pharmacy, Twentieth Edition, Lippincott Williams & Wilkins; 20th edition (Dec. 15, 2000). Some solvents may also serve as solubilizing agents. Examples of solvents for use in the invention include Maisine^{™} 35-1 (glyceryl monolineate) that comprises long chain fatty acids, for example glyceryl linoleate, PEG400, PEG800, PEG 1200, PEG 3350, ethanol, glycerin, PPG, polysorbates, povidone (PVP), and Transcutol^{®} HP (glycol monoethyl ether).

Solvents that may be used include but are not limited to DMSO, ethanol, methanol, isopropyl alcohol, castor oil, propylene glycol, glycerin, polysorbate 80, benzyl alcohol, dimethyl acetamide (DMA), dimethyl formamide (DMF), triacetin, diacetin, corn oil, acetyl triethyl citrate (ATC), ethyl lactate, glycerol formal, ethoxy diglycol (Transcutol, Gattefosse), tryethylene glycol dimethyl ether (Triglyme), dimethyl isosorbide (DMI), γ-butyrolactone, N-Methyl-2-pyrrolidinone (NMP), polyethylene glycol of various molecular weights, including but not limited to PEG 300 and PEG 400, and polyglycolated capryl glyceride (Labrasol, Gattefosse), combinations of any one or more of the foregoing, or analogs or derivatives of any one or more of the foregoing.

In another aspect, the solvent is a polyethylene glycol. Polyethylene glycol is known by various names and is available in various preparations, including but not limited to macrogels, macrogel 400, macrogel 1500, macrogel 4000, macrogel 6000, macrogel 20000, macrogola, breox PEG; carbowax; carbowax sentry; Hodag PEG; Lipo; Lipoxol; Lutrol E; PEG; Pluriol E; polyoxyethylene glycol, and the like. For example, the polyethylene glycol is a liquid PEG, and is one or more of PEG 300, PEG 400, PEG 800, PEG 1200, PEG3350, PEG 6000, and the like.

Phospholipid solvents can also be used, such as lecithin, phosphatidylcholine, or a mixture of various diglycerides of stearic, palmitic, and oleic acids, linked to the choline ester of phosphoric acid, hydrogenated soy phosphatidylcholine (HSPC), distearoylphosphatidylglycerol (DSPG), L-α-dimyristoylphosphatidylcholine (DMPC), or L-α-dimyristoylphosphatidylglycerol (DMPG).

Further examples of solvents include, for example, components such as alcohols, propylene glycol, polyethylene glycol of various molecular weights, propylene glycol esters, propylene glycol esterified with fatty acids such as oleic, stearic, palmic, capric, linoleic, etc; medium chain mono-, di-, or triglycerides, long chain fatty acids, naturally occurring oils, and a mixture thereof. The oily components for the solvent system include commercially available oils as well as naturally occurring oils. The oils may further be vegetable oils or mineral oils. The oils can be characterized as non-surface active oils, which typically have no hydrophile lipophile balance value. Commercially available substances comprising medium chain triglycerides include, but are not limited to, Captex 100, Captex 300, Captex 355, Miglyol 810, Miglyol 812, Miglyol 818, Miglyol 829, and Dynacerin 660. Propylene glycol ester compositions that are commercially available encompass Captex 200 and Miglyol 840, and the like. The commercial product, Capmul MCM, comprises one of many possible medium chain mixtures comprising monoglycerides and diglycerides.

Other solvents include naturally occurring oils such as peppermint oil, and seed oils. Exemplary natural oils include oleic acid, castor oil, safflower seed oil, soybean oil, olive oil, sunflower seed oil, sesame oil, and peanut oil. Soy fatty acids may also be used. Examples of fully saturated non-aqueous solvents include, but are not limited to, esters of medium to long chain fatty acids (such as fatty acid triglycerides with a chain length of about C₆ to about C₂₄). Hydrogenated soybean oil and other vegetable oils may also be used. Mixtures of fatty acids may be split from the natural oil (for example coconut oil, palm kernel oil, babassu oil, or the like) and refined. In some embodiments, medium chain (about C₈ to about C₁₂) triglycerides, such as caprilyic/capric triglycerides derived from coconut oil or palm seed oil, may be used. Medium chain mono- and diglycerides may also be used. Other fully saturated non-aqueous solvents include, but are not limited to, saturated coconut oil (which typically includes a mixture of lauric, myristic, palmitic, capric and caproic acids), including those sold under the Miglyol^{™} and bearing trade designations 810, 812, 829 and 840). Non-aqueous solvents include isopropyl myristate. Examples of synthetic oils include triglycerides and propylene glycol diesters of saturated or unsaturated fatty acids having 6 to 24 carbon atoms such as, for example hexanoic acid, octanoic (caprylic), nonanoic (pelargonic), decanoic (capric), undecanoic, lauric, tridecanoic, tetradecanoic (myristic), pentadecanoic, hexadecanoic (palmitic), heptadecanoic, octadecanoic (stearic), nonadecanoic, heptadecanoic, eicosanoic, heneicosanoic, docosanoic and lignoceric acids, and the like. Examples of unsaturated carboxylic acids include oleic, linoleic and linolenic acids, and the like. The non-aqueous solvent can comprise the mono-, di- and triglyceryl esters of fatty acids or mixed glycerides and/or propylene glycol mono- or diesters wherein at least one molecule of glycerol has been esterified with fatty acids of varying carbon atom length. A non-limiting example of a "non-oil" useful as a solvent is polyethylene glycol.

Exemplary vegetable oils include cottonseed oil, corn oil, sesame oil, soybean oil, olive oil, fractionated coconut oil, peanut oil, sunflower oil, safflower oil, almond oil, avocado oil, palm oil, palm kernel oil, babassu oil, beechnut oil, linseed oil, rape oil and the like. Mono-, di-, and triglycerides of vegetable oils, including but not limited to corn, may also be used.

Polyvinyl pyrrolidone (PVP), cross-linked or not, may also be used as a solvent. Further solvents include but are not limited to C₆-C₂₄ fatty acids, oleic acid, Imwitor 742, Capmul, F68, F68 (Lutrol), PLURONICS including but not limited to PLURONICS F108, F127, and F68, Poloxamers, Tetronics, F127, cyclodextrins such as α-cyclodextrin,-cyclodextrin, hydroxypropyl- β -cyclodextrin, sulfobutylether- β -cyclodextrin (Captisol); CMC, polysorbitan 20, Cavitron, polyethylene glycol of various molecular weights including but not limited to PEG 300 and PEG 400. Beeswax and d-α-tocopherol (Vitamin E) may also be used as solvents.

In another aspect of the invention, the solvent can be N-methylpyrrolidone (NMP), dimethyl-acetamine (DMA), dimethyl sulfoxide (DMSO), propylene glycol (PG), polyethylene glycol 600 (PEG 600), polyethylene glycol 400 (PEG 400), ethanol, or a mixture of one or more thereof. For example, the solvent comprises a combination of solvents including N-methyl pyrrolidone (NMP), dimethyl-acetamine (DMA), or dimethyl sulfoxide (DMSO). Alternatively, the solvent comprises a combination of solvents including propylene glycol (PG), polyethylene glycol 600 (PEG 600), or polyethylene glycol 400 (PEG 400). In some aspects of the invention, the solvent can comprise a combination of at least two solvents.

In some aspects of the invention, the solvent is polyethoxylated castor oil (e.g., Cremophor (PEG 35 castor oil)), monoglycerides and/or diglycerides of caprylic acid (e.g., Capmul MCM (C8)), nonionic polymer of the alkyl aryl polyether alcohol (e.g., tyloxapol (ethoxylated p-tert-octylphenol formaldehyde polymer)), Phosal^{®} 50PG, ethanol, or a mixture of one or more thereof. In some aspects of the invention, the solvent can comprise a combination of at least two solvents. In some variations, the at least two solvents comprising a first solvent such as polyethoxylated castor oil (e.g., Cremophor (PEG 35 castor oil)) or nonionic polymer of the alkyl aryl polyether alcohol (e.g., tyloxapol (ethoxylated p-tert-octylphenol formaldehyde polymer)) and a second solvent such as monoglycerides and/or diglycerides of caprylic acid (e.g., Capmul MCM (C8)). In some aspects, the solvent may further comprise 50% phosphatidylcholine in propylene glycol/ethanol carrier (e.g., Phosal^{®} 50PG).

In some variations, the solvent is N-methylpyrrolidone (NMP), dimethyl-acetamine (DMA), dimethyl sulfoxide (DMSO), propylene glycol (PG), polyethylene glycol 600 (PEG 600), polyethylene glycol 400 (PEG 400), ethanol, or a mixture of one or more thereof. In some variations, the solvent comprises a combination of solvents including N-methyl pyrrolidone (NMP), dimethyl-acetamine (DMA), or dimethyl sulfoxide (DMSO). In one aspect of the invention, the solvent comprises a combination of solvents including propylene glycol (PG), polyethylene glycol 600 (PEG 600), or polyethylene glycol 400 (PEG 400). In another aspect of the invention, the solvent may comprise a combination of at least two solvents. In another aspect of the invention, the at least two solvents comprising a first solvent such as N-methylpyrrolidone (NMP), dimethyl-acetamine (DMA), or dimethyl sulfoxide (DMSO) and a second solvent such as propylene glycol (PG), polyethylene glycol 600 (PEG 600), or polyethylene glycol 400 (PEG 400).

In yet another aspect of the invention, the solvent is polyethoxylated castor oil (e.g., Cremophor (PEG 35 castor oil)), monoglycerides and/or diglycerides of caprylic acid (e.g., Capmul MCM (C8)), nonionic polymer of the alkyl aryl polyether alcohol (e.g., tyloxapol (ethoxylated p-tert-octylphenol formaldehyde polymer)), Phosal^{®} 50PG, ethanol, or a mixture of one or more thereof. The solvent can comprise a combination of at least two solvents. For example, the at least two solvents comprising a first solvent such as polyethoxylated castor oil (e.g., Cremophor (PEG 35 castor oil)) or nonionic polymer of the alkyl aryl polyether alcohol (e.g., tyloxapol (ethoxylated p-tert-octylphenol formaldehyde polymer)) and a second solvent such as monoglycerides and/or diglycerides of caprylic acid (e.g., Capmul MCM (C8)). The solvent may further comprise 50% phosphatidylcholine in propylene glycol/ethanol carrier (e.g., Phosal^{®} 50PG), and the solvent can further comprise ethanol.

### Surfactants

Surfactants that can be used may be determined by mixing a therapeutic agent of interest with a putative solvent and a putative surfactant, and observing the characteristics of the formulation after exposure to a medium. Many surfactants are possible. Combinations of surfactants, including combinations of various types of surfactants, can also be used. For example, surfactants which are nonionic, anionic (i.e. soaps, sulfonates), cationic (i.e. CTAB), zwitterionic, polymeric or amphoteric can be used.

Examples of some surfactants, mixtures, and other equivalent compositions having an hydrophilic-lipophilic balance (HLB) less than or equal to 10 are propylene glycols, glyceryl fatty acids, glyceryl fatty acid esters, polyethylene glycol esters, glyceryl glycol esters, polyglycolyzed glycerides and polyoxyethyl steryl ethers. Propylene glycol esters or partial esters form the composition of commercial products, such as Lauroglycol FCC, which contains propylene glycol laureate.

The surfactants or solubilizing agents that may be employed may be selected from solubilizing agents having a HLB of 8-18, HLB of 7-9 and HLB of 8-12, HLB of 13-15, polyoxyethanyl-tocopheryl-sebacate (PTS), polyoxyethanyl-sitosterol-sebacate (PSS), polyoxyethanyl-cholesterol-sebacate (PCS), polyoxyethanyl-ubiquinol-sebacate (PQS) and combinations or mixtures thereof. In one aspect, the above solubilizing agent is selected from the group consisting of Poloxamer 188, Polysorbate 80, Polysorbate 20, Vitamin E-TPGS, Solutol HS 15, PEG-40 Hydrogenated castor oil (Cremophor RH40), PEG-35 Castor oil (Cremophor EL), PEG-8-glyceryl capylate/caprate (Labrasol), PEG-32-g)yceryl laurate (Gelucire 44/14), PEG-32-glyceryl palmitostearate (Gelucire 50/13); Polysorbate 85, Polyglyceryl-6-dioleate (Caprol MPGO), Mixtures of high and low HLB emulsifiers; Sorbitan monooleate (Span 80), Capmul MCM, Maisine 35-1, Glyceryl monooleate, Glyceryl monolinoleate, PEG-6-glyceryl oleate (Labrafil M 1944 CS), PEG-6-glyceryl linoleate (Labrafil M 2125 CS), Oleic acid, Linoleic acid, Propylene glycol monocaprylate (e.g. Capmul PG-8 or Capryol 90), Propylene glycol monolaurate (e.g., Capmul PG-12 or Lauroglycol 90), Polyglyceryl-3 dioleate (Plurol Oleique CC497), Polyglyceryl-3 diisostearate (Plurol Diisostearique) and Lecithin with and without bile salts, or combinations thereof.

### Stabilizers

The formulations described herein may further comprise various other components such as stabilizers, for example. Stabilizers that may be used in the formulations described herein include but are not limited to agents that will (1) improve the compatibility of excipients with the encapsulating materials such as gelatin, (2) improve the stability (e.g. prevent crystal growth of a therapeutic agent such as tacrolimus or ascomycin) of a therapeutic agent such as tacrolimus ascomycin, or their prodrugs or derivatives, and/or (3) improve formulation stability. Note that there is overlap between components that are stabilizers and those that are solvents, solubilizing agents or surfactants, and the same component can carry out more than one role.

Stabilizers may be selected from fatty acids, fatty alcohols, alcohols, long chain fatty acid esters, long chain ethers, hydrophilic derivatives of fatty acids, polyvinylpyrrolidones, polyvinylethers, polyvinyl alcohols, hydrocarbons, hydrophobic polymers, moisture-absorbing polymers, and combinations thereof. Amide analogues of the above stabilizers can also be used. The chosen stabilizer may change the hydrophobicity of the formulation (e.g. oleic acid, waxes), or improve the mixing of various components in the formulation (e.g. ethanol), control the moisture level in the formula (e.g. PVP), control the mobility of the phase (substances with melting points higher than room temperature such as long chain fatty acids, alcohols, esters, ethers, amides etc. or mixtures thereof; waxes), and/or improve the compatibility of the formula with encapsulating materials (e.g. oleic acid or wax). Some of these stabilizers may be used as solvents/co-solvents (e.g. ethanol). Stabilizers may be present in sufficient amount to inhibit the active agent's crystallization.

Examples of stabilizers include, but are not limited to, saturated, monoenoic, polyenoic, branched, ring-containing, acetylenic, dicarboxylic and functional-group-containing fatty acids such as oleic acid, caprylic acid, capric acid, caproic acid, lauric acid, myristic acid, palmitic acid, stearic acid, behenic acid, linoleic acid, linolenic acid, eicosapentaenoic acid (EPA), DHA; fatty alcohols such as stearyl alcohol, cetyl alcohol, ceteryl alcohol; other alcohols such as ethanol, isopropyl alcohol, butanol; long chain fatty acid esters, ethers or amides such as glyceryl stearate, cetyl stearate, oleyl ethers, stearyl ethers, cetyl ethers, oleyl amides, stearyl amides; hydrophilic derivatives of fatty acids such as polyglyceryl fatty acids, polyethylene glycol fatty acid esters; polyvinylpyrrolidones, polyvinylalcohols (PVAs), waxes, docosahexaenoic acid and de-hydroabietic acid etc.

The stabilizer can be a cellulose derivative. Suitable cellulose derivatives include, for example, hydroxypropyl methyl cellulose (HPMC), ethyl cellulose (EC), hydroxyethyl cellulose (HEC), hydroxyethyl ethyl cellulose (HEEC), hydroxypropyl cellulose (HPC), methyl cellulose (MC) and mixtures thereof, preferably hydroxypropyl methyl cellulose.

### Bioavailability Enhancers

A "bioavialability enhancer" is an agent capable of enhancing bioavailability and bioefficacy of a particular drug with which it is combined, without any typical pharmacological activity of its own at the dose used. In one aspect, the formulation can contain one or more bioavailability enahncers. Capryol 90, Capryol PGMC, Lauroglycol 90 and Lauroglycol FCC can be used as propylene glycol esters that are bioavailability enhancers. Other propylene glycol esters or partial esters form the composition of commercial products, such as Lauroglycol FCC, which contains propylene glycol laureate, can also be used. Any of the bioavailability enhancers which are commonly used in the manufacture of pharmaceutically acceptable solid, liquid, or other dosage forms may also be used for the purposes of formulation,

### Plasticizer

In one aspect, the formulations of the invention can contain one or more plasticizers. A plasticizer is generally a high boiling point solid or liquid. Suitable plasticizers can be added from about 0.01% to about 50% by weight (w/w) of the coating composition. Plasticizers include, but are not limited to, diethyl phthalate, citrate esters, polyethylene glycol, glycerol, acetylated glycerides, triacetin, polypropylene glycol, polyethylene glycol, triethyl citrate, dibutyl sebacate, stearic acid, stearol, stearate, and castor oil. In one aspect, the plasticizer is trietylcitrate.

### Rate Controlling Excipients

In one aspect, the formulations described herein are formulated as enteric coated delayed release oral dosage forms, i.e., as an oral dosage form of a pharmaceutical composition as described herein which utilizes an enteric coating to affect release in the small intestine or large intestine. Any coatings can be applied to the softgel capsule to a sufficient thickness such that the entire coating does not dissolve in the gastrointestinal fluids at pH below about 5, but does dissolve at pH about 5 and above.

The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). Furthermore, a time delay material such as, e.g., glyceryl monostearate or glyceryl distearate may be employed.

In accordance with the practice of the invention, examples of a rate controlling excipient include, but are not limited to, hydroxypropyl cellulose, hypromellose, ethyl cellulose, and prop-2-enoic acid. One suitable example of a prop-2-enoic acid is Carbopol^{®} (Noveon or Dow Chemical Co.). Examples of delay release polymers include a neutral methacrylic polymer such Eudragit^{®} FS30D, Eudragit^{®} S100, Eudragit^{®} L100-55 and/or any mixture or combination thereof (Rohm). Eudragit^{®} L100-55 is an enteric polymer which can be used in coated dosage forms to target the drug release in the upper small intestine where the pH is above 5.5. Eudragit^{®} S100 can be used to achieve targeted drug release in the lower small intestine to the colon, where the pH is above 7. The modified release components of the formulations of this invention can be formulated with any, and/or a mixture, of the above polymers, to achieve the desired plasma concentration profiles. The choice of the polymers that can be used in the invention includes, but is not limited to, Eudragit^{®}, cellulose acetate phthalate, polyvinyl acetate phthalate, hydroxypropyl methylcellulose phthalate, hydroxypropyl methylcellulose acetate succinate LF, hydroxypropyl methylcellulose acetate succinate HF, and others.

Conventional coating techniques such as spray or pan coating are employed to apply coatings. The coating thickness must be sufficient to ensure that the oral dosage form remains intact until the desired site of topical delivery in the intestinal tract is reached,

The foregoing ranges are merely suggestive, as the number of variables in regard to an individual treatment regime is large, and considerable excursions from these recommended values are not uncommon.

### Methods of use

A compound of the invention, such as ascomycin, or a pharmaceutically acceptable solvate, salt, analog, or prodrug thereof, can be administered to a subject upon determination of the subject as having pulmonary hypertension, in particular pulmonary arterial hypertention, or unwanted condition that would benefit by treatment with said compound. The determination can be made by medical or clinical personnel as part of a diagnosis of a disease or condition in a subject.

For administration to non-human animals, the drug or a pharmaceutical composition containing the drug may also be added to the animal feed or drinking water. It will be convenient to formulate animal feed and drinking water products with a predetermined dose of the drug so that the animal takes in an appropriate quantity of the drug along with its diet. It will also be convenient to add a premix containing the drug to the feed or drinking water approximately immediately prior to consumption by the animal.

### Kits/Articles of manufacture

For use in the therapeutic applications described herein, kits and articles of manufacture are also within the scope of the invention. Such kits can comprise a carrier, package, or container that is compartmentalized to receive one or more containers such as vials, tubes, and the like, each of the container(s) comprising one of the separate elements to be used in a method of the invention. Suitable containers include, for example, bottles, vials, syringes, and test tubes. The containers can be formed from a variety of materials such as glass or plastic.

For example, the container(s) can comprise one or more compounds of the invention, optionally in a composition or in combination with another agent as disclosed herein. The container(s) optionally have a sterile access port (for example the container can be an intravenous solution bag or a vial having a stopper pierceable by a hypodermic injection needle). Such kits optionally comprising a compound with an identifying description or label or instructions relating to its use in the methods of the present invention.

A kit of the invention will typically may comprise one or more additional containers, each with one or more of various materials (such as reagents, optionally in concentrated form, and/or devices) desirable from a commercial and user standpoint for use of a compound of the invention. Non-limiting examples of such materials include, but not limited to, buffers, diluents, filters, needles, syringes; carrier, package, container, vial and/or tube labels listing contents and/or instructions for use, and package inserts with instructions for use. A set of instructions will also typically be included.

A label can be on or associated with the container. A label can be on a container when letters, numbers or other characters forming the label are attached, molded or etched into the container itself; a label can be associated with a container when it is present within a receptacle or carrier that also holds the container, e.g., as a package insert. A label can be used to indicate that the contents are to be used for a specific therapeutic application. The label can also indicate directions for use of the contents, such as in the methods described herein.

The terms "kit" and "article of manufacture" may be used as synonyms.

Having now generally described the invention, the same will be more readily understood through reference to the following examples which are provided by way of illustration, and are not intended to be limiting of the present invention, unless specified.

### EXAMPLES

Below are examples of specific embodiments for carrying out the present invention. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

### EXAMPLE 1

### Solubility

The solubility of tacrolimus was obtained in Maisine^{®} 35-1, sesame oil, Miglyol^{®} 812, Caspmul MCM EP, Labrafil M 2125 CS, peceol, Capryol 90, Lauroglycol FCC, Span 80, caprylic acid, Transcutol HP, Tween 80 (polysorbate 80), Kolliphor EL (Cremophor EL), labrasol, Vitamin E TPGS, PEG 400, propylene glycol, ethanol, Phosal 50 PG, triethylcitrate, and PEG 3350. In general, tacrolimus (~15mg) was added to approximately 250 mg of the vehicles. After the first addition of the API, the mixtures were shaken in a temperature-controlled vortex mixer for 24 hours at 25°C (50°C for Vitamin E TPGS and 60°C for PEG 3350) then examined for solid residues. If no residue was observed, further addition of API was performed until the total API added was ~55mg. After the final addition, samples were further shaken at temperature for at least 24 h. After the incubation, each sample was examined visually for solid residues. Suspensions were filtered using a centrifuge tube with 0.45 µm PVDF membrane filter (Millipore Durapore^{®}). The thick filtrate was weighed in to a 5-mL volumetric flask and diluted to mark with the HPLC diluent. Biphasic mixtures were vialed, spun on a centrifuge, and the clear solution was separated from the oily phase. All samples were prepared protected from light. Tacrolimus exhibited solubility of >56mg of API per gram of solvent in Caspmul MCM EP, Capryol 90, caprylic acid, labrasol, Vitamin E TPGS, PEG 400, propylene glycol, ethanol, Phosal 50 PG, triethylcitrate, and PEG 3350.

### EXAMPLE 2

### Immediate release capsule A (IR capsule A)

Tacrolimus (2 mg, 0.7% wt%) was dissolved in labrasol (99.26 wt%) and BHT was added (0.04 wt%). The solution was filled into a size 5 oval softgel capsule.

### EXAMPLE 3

### Immediate release capsule B (IR capsule B)

Tacrolimus (2 mg, 0.7% wt%) was dissolved in labrasol (93.26 wt%) and triethyl citrate (6% wt%) and BHT was added (0.04 wt%). The solution was filled into a size 5 oval softgel capsule.

### EXAMPLE 4

### Enteric Coating of immediate release capsule of Example 2. (ER capsule I)

Tacrolimus (2 mg, 0.7% wt%) was dissolved in labrasol (99.26 wt%) and BHT was added (0.04 wt%). The solution was filled into a size 5 oval softgel capsule, as described in Example 2. The softgel capsule was coated with ethylcellulose.

### EXAMPLE 5

### In Vivo Test of Immediate Release Formulations

*In vivo* studies to determine the PK profile of the compositions of the present invention relative to the PK profile of the commercially available tacrolimus product, i.e. Prograf^{®} was performed using Beagle dogs. Male Beagle dogs each having a body weight of 12-18 kg (starting weight) were used. The studies were conducted as open label, non-randomized, cross-over studies. Each dog was dosed with the specified dose of tacrolimus without taking the weight of the dog into consideration.

Blood samples were collected at vena jugularis externa at the following points of time: Pre-dose, 0.25h, 0.5h, 0.75h, 1, 2, 3, 4, 6, 8, 12 and 24 hours after dosing. 4 ml of blood were collected, mixed with EDTA, and the samples were frozen (-80 °C.). The blood samples were analyzed using on-line extraction LC/MS. The individual pharmacokinetic parameters were estimated by non-compartmental analysis, using Excel. Values for Cmax (maximum blood concentration), C(t) (concentration at time postdose) and Tmax (time to maximum blood concentration) were determined directly from the plasma concentration-time profiles. Values for AUC(t1-t2) (area under the blood curve from time 1 to time 2) were calculated by linear trapezoidal rule from time t1 to t2.

In the first part of the study, the dogs were fasted overnight prior to dosing with access to food returned approximately 2 hours post dose administration. The animals were dosed with the immediate release (IR) capsule A prepared in Example 2, IR capsule B prepared in Example 3, or commercially available Prograf^{®} or its generic. The PK data is presented in Table 1 below:

**Table 1: PK data from fasted Beagle dogs using immediate release formulations of Examples 2 and 3.**

| PK parameter | IR capsule A | IR capsule B | Prograf^{®} |
|---|---|---|---|
| Tmax (h) | 0.42 | 0.58 | 0.75 |
| Cmax (ng/mL) | 7.77 | 6.87 | 6.38 |
| AUC (ng^{∗}h/mL) | 27.3 | 26.8 | 25.3 |

In the second part of the study, dogs were fed prior to dosing, and the blood samples were collected as described above. The PK data is presented in Table 2 below:

**Table 2: PK data from fed Beagle dogs using immediate release formulations of Examples 2 and 3.**

| PK parameter | IR capsule A | IR capsule B | Prograf^{®} |
|---|---|---|---|
| Tmax (h) | 0.5 | 1.0 | 0.5 |
| Cmax (ng/mL) | 2.36 | 1.52 | 2.56 |
| AUC (ng^{∗}h/mL) | 9.90 | 9.16 | 7.52 |

In the third part of the study, the fasted dogs were given an anti-emetic with metoclopramide (0.5 mg/kg) via intramuscular injection approximately 60 minutes prior to dosing. The PK data is presented in Table 3 below.

**Table 3: PK data from fasted Beagle dogs using immediate release formulation of Example 2.**

| PK parameter | IR capsule A |
|---|---|
| Tmax (h) | 0.52 |
| Cmax₀₋₂₄ₕ (ng/mL) | 7.37 |
| AUC₀₋₂₄ₕ (ng^{∗}h/mL) | 27.8 |

The data show that the immediate release formulations of capsule A and capsule B are essentially equivalent to the marketed formulation but have improved bioavailability as measured by AUC₀₋₂₄ₕ. The food effect is notable and is shown to reduce both the AUC and Cmax, when comparing Table 1 (fasted) vs. Table 2 (fed). IR capsule A and IR capsule B also provide a good chemical stability of tacrolimus.

### EXAMPLE 6

### In Vivo Test of Extended Release Formulation

The study was conducted as outlined in Example 5. A single Beagle dog was fasted overnight prior to dosing with access to food returned approximately 2 hours post dose administration. The fasted dogs were given an anti-emetic with metoclopramide (0.5 mg/kg) via intramuscular injection approximately 60 minutes prior to dosing. The animals were dosed with the extended release (ER) capsule prepared in Example 4. The PK data is presented in Table 4 below:

**Table 4: PK data from fasted Beagle dogs using extended release formulation of Examples 4.**

| PK parameter | ER capsule I |
|---|---|
| Tmax (h) | 2.0 |
| Cmax (ng/mL) | 2.77 |
| AUC (ng^{∗}h/mL) | 11.1 |

The data show that the extended release formulation of enteric coated softgel capsule of ER capsule I has improved bioavailability as measured by AUC₀₋₂₄ₕ, and about 4-fold longer Tmax when compared with those results of the commercial product Prograf^{®} (see Table 2).

While the invention has been particularly shown and described with reference to a preferred embodiment and various alternate embodiments, it will be understood by persons skilled in the relevant art that various changes in form and details can be made therein without departing from the spirit and scope of the invention. All printed patents and publications referred to in this application are hereby incorporated herein in their entirety by this reference.

Clauses:
1. A soft gelatin capsule comprising:
   a shell and a liquid fill material wherein the liquid fill material comprises an active agent, or a pharmaceutically acceptable salt, solvate, analog, or prodrug thereof, dissolved in a solvent, wherein the active agent is an ascomycin class compound.
2. The soft gelatin capsule of clause 1, wherein the active agent is tacrolimus (FK-506), ascomycin (FK-520), pimecrolimus (33-epi-chloro-33-desoxy-ascomycin), ABT-281, SDZ 281-240, desmethyl ascomycin (FK-523), prolytacrolimus(FK-525), or combinations thereof.
3. The soft gelatin capsule of clause 1, wherein the daily dose provides whole blood concentration of about 0.02 ng/mL to about 50 ng/mL.
4. The soft gelatin capsule of clause 1, wherein the active agent is tacrolimus, ascomycin, desmethyl ascomycin, or prolyltacrolimus.
5. The soft gelatin capsule of clause 1, wherein the solvent is Maisine 35-1, sesame oil, miglyol 812, Capryol 90, Lauroglycol FCC, Span 80, caprylic acid, Transcutol HP, Tween 80 (polysorbate 80), Kolliphor EL (Cremophor EL), labrasol, Vitamin E TPGS, PEG 400, propylene glycol, ethanol, Phosal 50 PG, triethylcitrate, or PEG 3350.
6. The soft gelatin capsule of clause 5, wherein the solvent is labrasol, Capmul or combinations thereof.
7. The soft gelatin capsule of clause 1, wherein the solvent comprises a surfactant. The
8. soft gelatin capsule of clause 7, wherein the surfactant is Caspmul MCM EP, Labrafil M 2125 CS, peceol, Tween 80 (polysorbate 80), Kolliphor EL (Cremophor EL), labrasol, Vitamin E TPGS, or any combination thereof.
9. The soft gelatin capsule of clause 1, wherein the solvent further comprises a plasticizer.
10. The soft gelatin capsule of clause 9, wherein the plasticizer is triethylcitrate.
11. The soft gelatin capsule of clause 1, wherein the solvent comprises labrasol, triethyl citrate, and BHT.
12. The soft gelatin capsule of clause 1, which is coated with an enteric coating selected from the group consisting of: ethylcellulose, methacrylic acid copolymer, cellulose acetate phthalate, hydroxypropyl methylcellulose phthalate, hydroxypropyl methylcellulose acetate succinate, polyvinyl acetate phthalate, shellac, and any combination thereof.

## Claims

1. A capsule comprising tacrolimus or a pharmaceutically acceptable salt thereof, and a solubilizing agent and/or surfactant, wherein the solubilizing agent and/or surfactant comprises PEG-32-glyceryl palmitostearate (Gelucire 50/13).

2. The capsule of claim 1, wherein the capsule comprises a release rate controlling excipient.

3. The capsule of claim 2, wherein the release rate controlling excipient comprises glyceryl distearate.

4. The capsule of any one of claims 1 to 3, wherein the capsule further comprises a solvent.

5. The capsule of claim 4, wherein the solvent comprises butylated hydroxytoluene (BHT).

6. The capsule of any one of claims 1 to 5, wherein the solvent further comprises any one of polysorbates or macrogel 1500.

7. The capsule of any one of claims 1 to 6, wherein the capsule further comprises a stabilizer.

8. The capsule of any one of claims 1 to 7, wherein the capsule further comprises an organic acid, optionally wherein the capsule further comprises tartaric acid.

9. The capsule of any one of claims 1 to 8, wherein the capsule is a soft capsule.

10. The capsule of any one of claims 1 to 9, wherein the capsule further comprises wetting or emulsifying agents, pH buffering agents, a co-solvent, a lipophilic surfactant, a hydrophilic surfactant, a plasticizer, a bioavailability enhancer, or a fatty acid.

11. The capsule of any one of any one of claims 1 to 10, wherein the capsule is for use as a medicament.

12. The composition of any one of claims 1 to 10, wherein the capsule is for use in the treatment or prevention of peripheral arterial hypertension (PAH).

13. The composition for use of claim 12, wherein the treatment of PAH includes the treatment of idiopathic PAH; familial PAH; PAH associated with a collagen vascular disease selected from:
scleroderma, CREST syndrome, systemic lupus erythematosus (SLE), rheumatoid arthritis, Takayasu's arteritis, polymyositis, and dermatomyositis; PAH associated with a congenital heali disease selected from: atrial septic defect (ASD), ventricular septic defect (VSD) and patent ductus arteriosus in an individual; PAH associated with portal hypertension; PAH associated with HIV infection; PAH associated with ingestion of a drug or toxin; P AH associated with hereditary haemorrhagic telangiectasia; P AH associated with splenectomy; P AH associated with significant venous or capillary involvement; PAH associated with pulmonary veno-occlusive disease (PVOD); and
P AH associated with pulmonary capillary hemangiomatosis (PCH).
